(19) Europäisches Patentamt
European Patent Office
Office européen des brevets

(11) Veröffentlichungsnummer: **0 013 542 B1**

(12) # EUROPÄISCHE PATENTSCHRIFT

(45) Veröffentlichungstag der Patentschrift: **02.06.82**

(51) Int. Cl.³: **C 07 D 307/32**, C 07 D 405/12, A 01 N 43/08, A 01 N 43/40

(21) Anmeldenummer: **79810190.3**

(22) Anmeldetag: **28.12.79**

(54) **Alpha-Phenoxy-propionsäure-gamma-butyrolactonester und -thioester mit herbizider Wirkung, deren Herstellung,sie als Wirkstoffe enthaltende Mittel und deren Verwendung.**

(30) Priorität: **04.01.79 CH 52/79**

(43) Veröffentlichungstag der Anmeldung: **23.07.80 Patentblatt 80/15**

(45) Bekanntmachung des Hinweises auf die Patenterteilung: **02.06.82 Patentblatt 82/22**

(84) Benannte Vertragsstaaten: **BE CH DE FR GB IT NL**

(56) Entgegenhaltungen:
**DE-A-2 433 067**
**DE-A-2 546 251**
**DE-A-2 804 074**

(73) Patentinhaber: **CIBA-GEIGY AG, Patentabteilung Postfach, CH-4002 Basel (CH)**

(72) Erfinder: **Böhner, Beat, Dr., Hügelweg 3, CH-4102 Binningen (CH)**
Erfinder: **Rempfler, Hermann, Dr., Brücklismattstrasse 16, CH-4107 Ettingen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 013 542 B1

BUNDESDRUCKEREI BERLIN

# α-Phenoxy-propionsäure-γ-butyrolactonester und -thioester mit herbizider Wirkung, deren Herstellung, sie als Wirkstoffe enthaltende Mittel und deren Verwendung

Die vorliegende Erfindung betrifft neue α-Phenoxy-propionsäure-γ-butyrolactonester und -thioester mit herbizider Wirkung, Verfahren zu deren Herstellung, Mittel welche sie als Wirkstoffe enthalten sowie deren Verwendung zur Bekämpfung unerwünschten Pflanzenwuchses.

Die α-Phenoxy-propionsäure-γ-butyrolactonester und -thioester entsprechen der Formel I

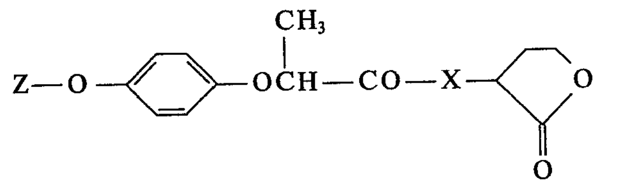

(I)

worin

X Sauerstoff oder Schwefel und
Z den 4-Trifluormethylphenyl- oder den 3,5-Dichlorpyridyl-2-rest

bedeuten.

Phenoxy-phenoxy-alkancarbonsäure-γ-butyrolactonester mit herbizider Wirkung sind in der DOS 2 804 074 beschrieben, andere Ester in der DOS 2 433 067. Ähnliche Pyridyloxy-phenoxy-propionsäureester sind aus der DOS 2 546 251 bekannt.

Die Verbindungen vorliegender Anmeldung sind neu und besitzen überraschenderweise ausgezeichnete von den Estern der obgenannten Offenlegungsschriften nicht erreichte Herbizidwirkung mit interessanten Selektivitäten für einzelne Kulturpflanzungen wie z. B. Soja, Baumwolle und Zuckerrüben, worin die grasartigen Unkräuter unter Kontrolle gehalten werden.

Die Verbindungen sind von geringer Toxizität für Menschen und Tiere. Ihre Handhabung kann ohne besondere Vorsichtsmaßnahmen erfolgen. Im Feld werden sie vorteilhafterweise in Aufwandmengen von 5 kg/ha und weniger eingesetzt. Sie können im Vorauflaufverfahren eingesetzt werden, bevorzugt ist jedoch der Einsatz nach dem Auflaufen der Kultur.

Die Herstellung der α-Phenoxy-propionsäure-γ-butyrolactonester und -thioester der Formel I erfolgt in an sich bekannter Weise durch Umsetzen der α-[4-(para-Trifluormethylphenoxy)-phenoxy]-propionsäure oder -thiopropionsäure respektive der α-[4(3',5'-Dichlorpyridyl-2'-oxy)-phenoxy]-propionsäure- oder thiopropionsäure der Formel II

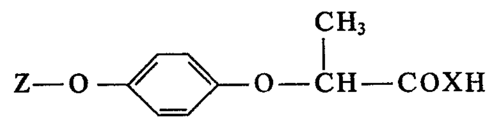

(II)

worin X und Z die unter Formel I gegebene Bedeutung haben, mit α-Brom-γ-butyrolacton der Formel III

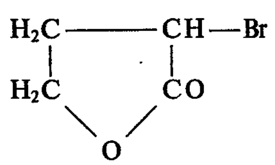

(III)

in einem inerten Lösungsmittel in Gegenwart eines säurebindenden Mittels.

Die als Ausgangsprodukte der Formel II benötigten α-Phenoxy-propionsäuren und -thiopropionsäuren sind bekannt. Ihre Herstellung ist z. B. in folgenden Publikationen beschrieben: DOS 2 531 643, DOS 2 546 251, GB-PS 1 507 643, US-PS 4 046 553 und CH-Patentanmeldung 14 398/77. α-Brom-γ-butyrolacton ist im Handel erhältlich.

Die folgenden Beispiele zeigen die Herstellung der erfindungsgemäßen Verbindungen. Temperaturen sind in Celsiusgraden angegeben, Teile und Prozentangaben beziehen sich auf das Gewicht.

Beispiel 1

2-[4-(4-Trifluormethylphenoxy)-phenoxy]-propionsäure-2-γ-butyrolacton-ester

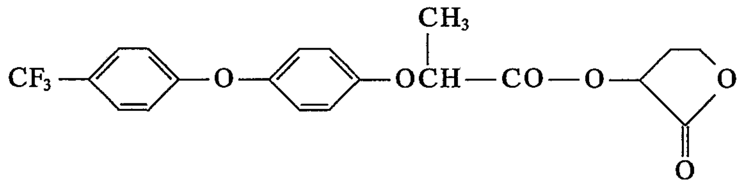

16,3 g (0,05 M) 2-[4-(4-Trifluormethylphenoxy)-phenoxy]-propionsäure werden in 30 ml Aceton gelöst und mit 8,2 g (0,06 M) Kaliumcarbonat versetzt. Man läßt 1 Stunde bei 40° ausrühren und gibt dann 4,9 ml (0,052 M) α-Brom-γ-butyrolacton zu. Nach 2 Stunden rühren bei 40° filtriert man das Reaktionsgemisch ab und dampft das Filtrat ein. Dieses löst man in wenig Tetrachlorkohlenstoff und behandelt die Lösung mit Aktivkohle. Nach dem Eindampfen erhält man ein braunes, klares Öl. Es sind 18,8 g (91,7% der Theorie) vom obengenannten Ester, welcher den Brechungsindex $n_D^{22}$ 1.5213 hat.

Beispiel 2

2-[4-(4-Trifluormethylphenoxy)-phenoxy]-thiopropionsäure-S-2-γ-butyrolacton-ester

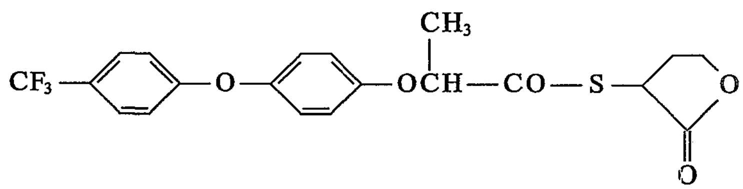

17,1 g (0,05 M) 2-(4-(4-Trifluormethylphenoxy)-phenoxy-propionthiolsäure werden in 30 ml Aceton gelöst und mit 8,2 g (0,06 M) Kaliumcarbonat versetzt. Die Reaktion verläuft leicht exotherm. Man läßt 30 Minuten bei 40° ausrühren. Dann tropft man bei Raumtemperatur 4,9 ml (0,052 M) α-Brom-γ-butyrolacton zu, was eine mäßig exotherme Reaktion zur Folge hat. Nach 10 Minuten bei 40° wird abfiltriert und das Filtrat eingedampft. Man erhält eine schwarze, viskose Masse. Diese löst man in wenig Tetrachlorkohlenstoff und behandelt die Lösung mit Aktivkohle. Nach dem Filtrieren und Eindampfen des Filtrats liegt das Produkt als hellgelbes, klares, leicht viskoses Öl vor, das rein ist im Dünnschichtchromatogramm. Es sind 17,4 g (81,7% der Theorie) des obigen Esters, welcher einen Brechungsindex $n_D^{22}$ 1.5341 aufweist.

Beispiel 3

2-[4-(3',5'-Dichlorpyridyl-2'-oxy)-phenoxy)-propionsäure-2-γ-butyrolacton-ester

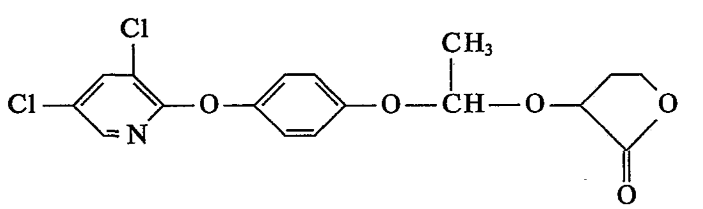

16,4 g (0,05 M) 2-(4-(3',5'-Dichlorpyridyl-2'-oxy)-phenoxy)-propionsäure werden in 30 ml Aceton gelöst und mit 8,2 g (0,06 M) Kaliumcarbonat versetzt. Man läßt 10 Minuten lang bei 40° ausrühren und tropft bei Raumtemperatur 4,9 ml (0,052 M) α-Brom-γ-butyrolacton zu. Dann wird das Reaktionsgemisch 1 Stunde bei 40° ausgerührt und abfiltriert. Das Filtrat behandelt man mit Aktivkohle und dampft es ein. Als Rückstand bleibt ein hellgelbes, klares, leicht viskoses Öl, das im Dünnschichtchromatogramm rein erscheint mit dem Brechungsindex $n_D^{22}$ 1.5549. Die Ausbeute beträgt 17,9 g (86,9% der Theorie).

## Beispiel 4

2-[4-(3′,5′-Dichlorpyridyl-2′-oxy)-phenoxy]-thiopropionsäure-S-2-γ-butyrolacton-ester

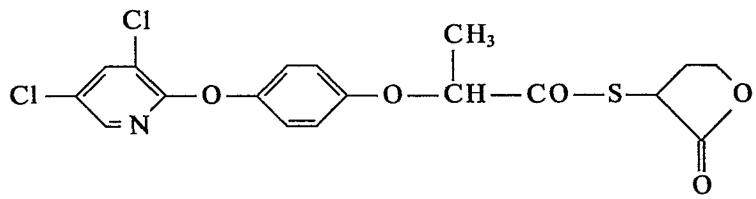

17,2 g (0,05 M) 2-(4-(3′,5′-Dichlorpyridyl-2′-oxy)-phenoxy)-propionthiolsäure werden in 30 ml Aceton gelöst und mit 8,2 g (0,055 M) Kaliumcarbonat versetzt. Die Reaktion ist leicht exotherm (32°). Man läßt noch 30 Minuten bei 40° ausrühren und tropft dann bei Raumtemperatur 4,9 ml (0,052 M) α-Brom-γ-butyrolacton zu, was eine mäßig exotherme Reaktion verursacht. Dann wird 30 Minuten bei 40° ausgerührt. Das Reaktionsgemisch wird zu einer blaugrauen Suspension. Man filtriert über Hyflo ab und erhält nach dem Eindampfen eine schwarze Masse. Diese löst man in wenig Tetrachlorkohlenstoff, wobei schwarze Flocken ausfallen. Diese werden abfiltriert und das Filtrat wird eingedampft. Es verbleibt ein hellgelber klarer, leicht viskoser Rückstand, 17,0 g (79,4% der Theorie) welcher im Dünnschichtchromatogramm rein erscheint und einen Brechungsindex $n_D^{22}$ 1.5840 aufweist.

Die neuen α-Phenoxy-propionsäure-oxofurylester und -thioester der Formel I sowie die sie enthaltenden Mittel besitzen selbst bei kleinen Aufwandmengen noch eine ausgezeichnete selektiv-herbizide Wirkung gegen Unkräuter in verschiedenen Kulturen, vorzugsweise in dikotylen Kulturen.

Ein bevorzugtes Einsatzgebiet ist z. B. die Bekämpfung von grasartigen Unkrautarten in Kulturen wie Baumwolle, Zuckerrüben, Soja und Gemüsekulturen.

Obwohl die neuen Wirkstoffe der Formel I bei pre- und post-emergenter Anwendung wirksam sind, scheint die post-emergenter Applikation als Kontaktherbizide den Vorzug zu verdienen, obwohl auch der pre-emergente Einsatz von Interesse ist.

Bevorzugt werden die neuen Wirkstoffe als z. B. 25%ige Spritzpulver oder z. B. 20%ige emulgierbare Konzentrate formuliert und mit Wasser verdünnt, auf die Pflanzenbestände post-emergent appliziert.

### Herbizide Wirkung bei Applikation der Wirkstoffe nach dem Auflaufen der Pflanzen (post-emergent)

Verschiedene Kulturpflanzen und Unkräuter werden aus den Samen in Töpfen im Gewächshaus aufgezogen, bis sie das 4- bis 6-Blatt-Stadium erreicht haben. Dann werden die Pflanzen mit wässerigen Wirkstoffemulsionen (erhalten aus einem 20%igen emulgierbaren Konzentrat) in verschiedenen Dosierungen gespritzt. Die behandelten Pflanzen werden dann bei optimalen Bedingungen von Licht, Begießung, Temperatur (22–25°C und Luftfeuchtigkeit 50–70% relativ) gehalten. 15 Tage nach Behandlung erfolgte die Auswertung des Versuches gemäß folgender Bewertungskala:

1 Pflanze abgestorben
2–8 Zwischenstufen der Schädigung
9 Pflanze gedeiht wie unbehandelte Kontrolle

Die Verbindungen vorliegender Erfindung sind in diesem Versuch mit dem aus der DOS No. 2 804 074 bekannten 2-[4-(4-Chlorphenoxy)-phenoxy]-propionsäure-2-γ-butyrolactonester = Verbindung A verglichen worden. Die Ergebnisse sind in der untenstehenden Tabelle festgehalten.

| Geprüfte Verbindung | Beispiel 1 | | | | | Beispiel 2 | | | | | Beispiel 3 | | | | | Beispiel 4 | | | | | Beispiel A | | | | |
|---|---|---|---|---|---|---|---|---|---|---|---|---|---|---|---|---|---|---|---|---|---|---|---|---|---|
| Aufwandmenge in gk/ha | 2 | 1 | $\frac{1}{2}$ | $\frac{1}{4}$ | $\frac{1}{8}$ | 2 | 1 | $\frac{1}{2}$ | $\frac{1}{4}$ | $\frac{1}{8}$ | 2 | 1 | $\frac{1}{2}$ | $\frac{1}{4}$ | $\frac{1}{8}$ | 2 | 1 | $\frac{1}{2}$ | $\frac{1}{4}$ | $\frac{1}{8}$ | 2 | 1 | $\frac{1}{2}$ | $\frac{1}{4}$ | $\frac{1}{8}$ |
| Pflanze | | | | | | | | | | | | | | | | | | | | | | | | | |
| Soja | 7 | 8 | 9 | 9 | 9 | 7 | 9 | 9 | 9 | 9 | 7 | 8 | 9 | 9 | 9 | 5 | 8 | 9 | 9 | 9 | 9 | 9 | 9 | 9 | 9 |
| avena fatua | 1 | 1 | 3 | 9 | 9 | 2 | 2 | 6 | 9 | 9 | 1 | 1 | 1 | 3 | 3 | 1 | 1 | 2 | 4 | 4 | 9 | 9 | 9 | 9 | 9 |
| lolium perenne | 2 | 3 | 4 | 6 | 9 | 2 | 3 | 4 | 4 | 9 | 2 | 3 | 3 | 6 | 7 | 2 | 2 | 3 | 3 | 8 | 4 | 6 | 6 | 9 | 9 |
| alopecurus myosuroides | 2 | 2 | 5 | 6 | 9 | 2 | 3 | 3 | 6 | 7 | 2 | 2 | 3 | 3 | 4 | 2 | 2 | 3 | 3 | 4 | 2 | 2 | 6 | 7 | 9 |
| digitaria sanguinalis | 1 | 1 | 1 | 2 | 2 | 1 | 1 | 1 | 2 | 2 | 1 | 1 | 1 | 1 | 1 | 1 | 1 | 1 | 2 | 2 | 1 | 2 | 3 | 3 | 4 |
| sorghum halepense | 1 | 1 | 1 | 1 | 5 | 1 | 1 | 1 | 1 | 2 | 1 | 1 | 1 | 1 | 1 | 1 | 1 | 1 | 1 | 1 | 7 | 9 | 9 | 9 | 9 |
| rottboellia exaltata | 1 | 1 | 2 | 4 | 9 | 1 | 2 | 2 | 3 | 9 | 1 | 1 | 1 | 1 | 3 | 1 | 1 | 1 | 1 | 6 | 6 | 8 | 9 | 9 | 9 |

Die neuen Wirkstoffe der Formel I sind stabile Verbindungen, welche in üblichen organischen Lösungsmitteln, wie Alkoholen, Äthern, Ketonen, Dimethylformamid, Dimethylsulfoxyd etc. löslich sind.

Die Herstellung erfindungsgemäßer Mittel erfolgt in an sich bekannter Weise durch inniges Vermischen und Vermahlen von Wirkstoffen der allgemeinen Formel I mit geeigneten Trägerstoffen und/oder Verteilungsmitteln, gegebenenfalls unter Zusatz von gegenüber den Wirkstoffen inerten Antischaum-, Netz-, Dispersions- und/oder Lösungsmitteln. Die Wirkstoffe können in den folgenden Aufarbeitungsformen vorliegen und angewendet werden:

Feste Aufarbeitungsformen:
Stäubemittel, Streumittel, Granulate, Umhüllungsgranulate, Imprägnierungsgranulate und Homogengranulate;
In Wasser dispergierbare Wirkstoffkonzentrate:
Spritzpulver (wettable powder), Paste, Emulsionen; Emulsionskonzentrate
Flüssige Aufarbeitungsformen:
Lösungen

Die Wirkstoffkonzentrationen betragen in den erfindungsgemäßen Mitteln 1 bis 80 Gewichtsprozent und können gegebenenfalls bei der Anwendung auch in geringen Konzentrationen wie etwa 0,05 bis 1% vorliegen.

Den beschriebenen erfindungsgemäßen Mitteln lassen sich andere biozide Wirkstoffe oder Mittel beimischen.

Die folgenden Formulierungsbeispiele sollen die Herstellung von festen und flüssigen Aufbereitungsformen näher erläutern.

## Emulsionskonzentrat

Zur Herstellung eines 25%igen Emulsionskonzentrates werden

25 Teile eines Wirkstoffes der Formel I,
5 Teile einer Mischung von Nonylphenolpolyoxyäthylen und Calciumdodecylbenzolsulfat,
15 Teile Cyclohexanon,
55 Teile Xylol

miteinander vermischt. Dieses Konzentrat kann mit Wasser zu Emulsionen auf geeigneten Konzentrationen von z. B. 0,1 bis 10% verdünnt werden. Solche Emulsionen eigenen sich zur Bekämpfung von Unkräutern in Kulturpflanzungen.

## Granulate

Zur Herstellung eines 5%igen Granulates werden die folgenden Stoffe verwendet:

5 Teile eines der Wirkstoffe der Formel I,
0,25 Teile Epichlorhydrin,
0,25 Teile Cetylpolyglykoläther,
3,50 Teile Polyäthylenglykol,
91 Teile Kaolin (Korngröße: 0,3 – 0,8 mm).

Die Aktivsubstanz wird mit Epichlorhydrin vermischt und in 6 Teilen Aceton gelöst, hierauf wird Polyäthylenglykol und Cetylpolyglykoläther zugesetzt. Die so erhaltene Lösung wird auf Kaolin aufgesprüht und anschließend im Vakuum verdampft.

## Spritzpulver

Zur Herstellung eines a) 70%igen und b) 10%igen Spritzpulvers werden folgende Bestandteile verwendet:

a) 70 Teile eines Wirkstoffes der Formel I,
5 Teile Natriumdibutylnaphtylsulfonat,
3 Teile Naphthalinsulfonsäuren-Phenolsulfonsäuren-Formaldehyd-Kondensat 3 : 2 : 1,
10 Teile Kaolin,
12 Teile Champagne-Kreide;

b) 10 Teile eines Wirkstoffes der Formel I,
3 Teile Gemisch der Natriumsalze von gesättigten Fettalkoholsulfaten,
5 Teile Naphthalinsulfonsäuren-Formaldehyd-Kondensat,
82 Teile Kaolin.

Der angegebene Wirkstoff wird auf die entsprechenden Trägerstoffe (Kaolin und Kreide) aufgezogen und anschließend mit den übrigen Bestandteilen vermischt und vermahlen. Man erhält Spritzpulver von vorzüglicher Benetzbarkeit und Schwebefähigkeit. Aus solchen Spritzpulvern können durch Verdünnen mit Wasser Suspensionen von 0,1 – 80% Wirkstoff erhalten werden, die sich zur Unkrautbekämpfung in Pflanzenkulturen eignen.

Paste

Zur Herstellung einer 45%igen Paste werden folgende Stoffe verwendet:

45 Teile eines Wirkstoffes der Formel I,
5 Teile Natriumaluminiumsilikat,
14 Teile Cetylpolyglykoläther mit 8 Mol Äthylenoxid,
1 Teil Oleylpolyglykoläther mit 5 Mol Äthylenoxid,
2 Teile Spindelöl,
10 Teile Polyäthylglykol,
23 Teile Wasser.

Der Wirkstoff wird mit den Zuschlagstoffen in dazu geeigneten Geräten innig vermischt und vermahlen. Man erhält eine Paste, aus der sich durch Verdünnen mit Wasser Suspensionen jeder gewünschten Konzentration herstellen lassen.

**Patentansprüche**

1. α-Phenoxy-propionsäure-γ-butyrolactonester und -thioester der Formel I

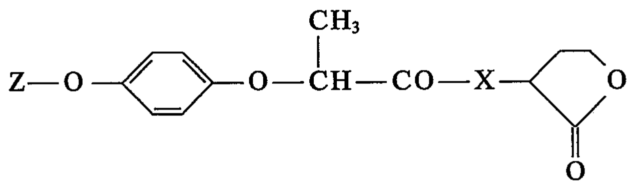

(I)

worin

X Sauerstoff oder Schwefel
Z der 4-Trifluormethylphenyl- oder 3,5-Dichlorpyridyl-2-rest

bedeuten.

2. Als Verbindung gemäß Anspruch 1, 2-[4-(4-Trifluormethylphenoxy)-phenoxy]-propionsäure-2-γ-butyrolacton-ester.

3. Als Verbindung gemäß Anspruch 1, 2-[4-(4-Trifluormethylphenoxy)-phenoxy]-thiopropionsäure-S-2-γ-butyrolacton-ester.

4. Als Verbindung gemäß Anspruch 1, 2-[4-(3′,5′-Dichlorpyridyl-2′-oxy)-phenoxy]-propionsäure-2-γ-butyrolacton-ester.

5. Als Verbindung gemäß Anspruch 1, 2-[4-(3′,5′-Dichlorpyridyl-2′-oxy)-phenoxy]-thiopropionsäure-S-2-γ-butyrolacton-ester.

6. Die Herstellung der α-Phenoxy-propionsäure-oxofurylester und -thioester der Formel I, Anspruch 1, dadurch gekennzeichnet, daß man in einem inerten Lösungsmittel in Gegenwart eines säurebindenden Mittels eine α-Phenoxy-propionsäure oder -thiosäure der Formel II

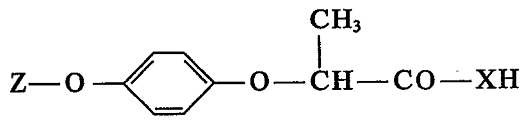

(II)

worin X und Z die unter Formel I, Anspruch 1, gegebene Bedeutung haben, mit α-Brom-γ-butyrolacton der Formel III

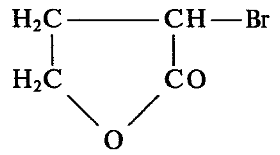

(III)

umsetzt.

7. Ein herbizides Mittel, welches neben inerten Zutaten als Wirkstoff einen α-Phenoxy-propionsäure-oxofurylester oder thioester der Formel I, Anspruch 1 enthält.

8. Die Verwendung der α-Phenoxy-propionsäure-oxofurylester oder -thioester der Formel I, Anspruch 1 oder sie enthaltende Mittel zur Bekämpfung unerwünschten Pflanzenwachstums.

9. Die Verwendung der α-Phenoxy-propionsäure-oxofurylester oder thioester der Formel I, Anspruch 1 oder sie enthaltender Mittel als selektive Herbizide in Kulturpflanzungen.

## Claims

1. An α-phenoxy-propionic acid-γ-butyrolactone ester or thioester of the formula I

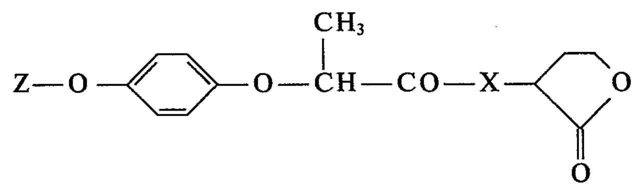

(I)

wherein

X is oxygen or sulfur,
Z is the 4-trifluoromethylphenoxy group or the 3,5-dichloropyridyl-2-oxy group.

2. As compound according to claim 1, 2-[4-(4-trifluoromethylphenoxy)-phenoxy]-propionic acid-2-γ-butyrolactone ester.

3. As compound according to claim 1, 2-[4-(4-trifluoromethylphenoxy)-phenoxy]-thiopropionic acid-S-2-γ-butyrolactone ester.

4. As compound according to claim 1, 2-[4-(3′,5′-dichloropyridyl-2′-oxy)-phenoxy]-propionic acid-2-γ-butyrolactone ester.

5. As compound according to claim 1, 2-[4-(3′,5′-dichloropyridyl-2′-oxy)-phenoxy]-thiopropionic acid-S-2-γ-butyrolactone ester.

6. The production of an α-phenoxy-propionic acid-oxofuryl ester or thioester of the formula I, claim 1, which process comprises reacting, in an inert solvent in the presence of an acid-binding agent, an α-phenoxy-propionic ot thiopropionic acid of the formula II

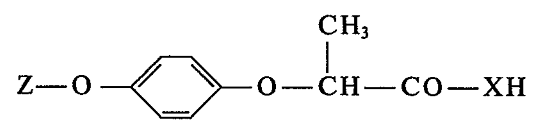

(II)

wherein X and Z have the meanings given under the formula I, claim 1, with α-bromo-γ-butyrolactone of the formula III

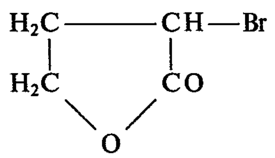

(III)

7. A herbicidal composition which contains as active substance an α-Phenoxy-propionic acid-oxofuryl ester or thioester of the formula I, claim 1, together with inert constituents.

8. The use of an α-phenoxy-propionic acid-oxofuryl ester or thioester of the formula I, claim 1, or of compositions containing it, for controlling undesirable plant growth.

9. The use of an α-phenoxy-propionic acid-oxofuryl ester or thioester of the formula I, claim 1, or of compositions containing it, as a selective herbicide in cultivated crops.

**Revendications**

1. γ-butyrolactonesters et -thioesters de l'acide α-phénoxy-propionique de formule I

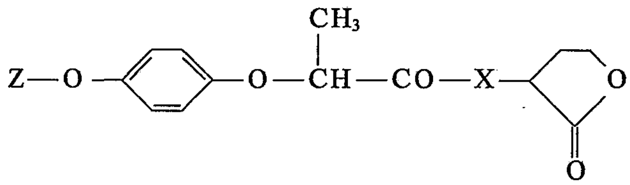

(I)

où

X représente un oxygène ou un soufre
Z représente le radical 4-trifluorométhylphényle ou le radical 3,5-dichloropyridyl-2.

2. Comme composé selon la revendication 1, le 2-γ-butyrolactone-ester de l'acide 2-[4-(4-trifluorométhylphénoxy)-phénoxy]-propionique.
3. Comme composé selon la revendication 1, le S-2-γ-butyrolactone-ester de l'acide 2-[4-(4-trifluorométhylphénoxy)-phénoxy]-thiopropionique.
4. Comme composé selon la revendication 1, le 2-γ-butyrolactone-ester de l'acide 2-[4-(3',5'-dichloropyridyl-2'-oxy)-phénoxy]-propionique.
5. Comme composé selon la revendication 1, le S-2-γ-butyrolactone-ester de l'acide 2-[4-(3',5'-dichloropyridyl-2'-oxy)-phénoxy]-thiopropionique.
6. La préparation des oxofurylesters et -thioesters de l'acide α-phénoxy-propionique de formule I selon la revendication 1, caractérisée en ce qu'on fait réagir dans un solvant inerte en présence d'un agent liant acide un acide α-phénoxy-propionique ou -thioïque de formule II

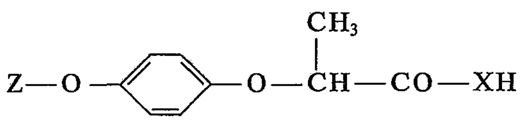

(II)

où X et Z ont la signification donnée pour la formule I selon la revendication 1, avec l'α-bromo-γ-butyrolactone de formule III

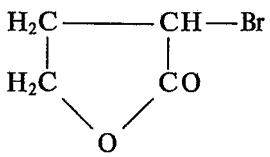

(III)

7. Agent herbicide contenant, outre des ingrédients inertes, comme matière active, un oxofurylester ou -thioester de l'acide α-phénoxy-propionique de formule I selon la revendication 1.
8. Application des oxofurylesters ou -thioesters de l'acide α-phénoxy-propionique de formule I de la revendication 1 ou des agents qui les contiennent à la lutte contre la croissance végétale indésirable.
9. Application des oxofurylesters ou -thioesters de l'acide α-phénoxy-propionique de formule I de la revendication 1 ou des agents qui les contiennent comme herbicides sélectifs dans les cultures.